# EUROPEAN PATENT SPECIFICATION

(11) **EP 1 241 225 B1**
(45) Date of publication and mention of the grant of the patent: **17.11.2004**
(21) Application number: 02005288.2
(22) Date of filing: 12.03.2002
(51) Int. Cl.: C08L 63/00, C08L 63/06, C08G 59/40, D21H 21/14

(54) **Resins acting as wet strength agents and creping aids and processes for preparing and using the same**
Harze verwendbar als Nassfestigkeitsmittel und Krepphilfsmittel, ihre Herstellung und ihre Verwendung
Résines agissant comme adjuvants de résistance à l'humidité et de crépage, procédés de leur préparation et leurs utilisations

(30) Priority: 12.03.2001 US 275158
(43) Date of publication of application: 18.09.2002
(73) Proprietor: HERCULES INCORPORATED, Wilmington, Delaware 19894-0001 (US)
(72) Inventor: Walton, Cynthia, London Grove, Pennsylvania 19348 (US); Warchol, Joseph F., Norristown, Pennsylvania 19403 (US)
(74) Representative: HOFFMANN - EITLE

(56) References cited:
- WO-A-01/38636
- WO-A-97/44519
- US-A- 5 660 687

## Description

### BACKGROUND OF THE INVENTION

### 1. FIELD OF THE INVENTION

The present invention relates generally to the process of manufacturing paper. Particularly, the present invention relates to resins and processes for preparing and using the same. More particularly, the present invention relates to the use of a polyepoxide, such as trigylcidylisocyanurate, in the production of epichlorohydrin-free resins used as wet strength agents and creping aids.

### 2. BACKGROUND OF THE INVENTION AND RELATED INFORMATION

The paper industry incorporates many compounds, including resins, into the process of manufacturing paper. Resins are commonly used within the industry as wet strength agents and as creping aids. These agents and aids are used in the manufacturing process in order to impart the desired characteristics of strength and softness to cellulosic products.

Cellulosic products, such as paperboards, tissue papers and writing papers are traditionally made by producing an aqueous slurry of cellulosic wood fibers, which may contain inorganic mineral extenders or pigments. The aqueous slurry is deposited on a moving wire or fabric, to facilitate the formation of a cellulose matrix. The cellulose matrix is then drained of excess water or liquids, pressed typically using felt, and dried into a final cellulosic product. In the preparation of a product such as tissue papers, wet strength agents may be added to enhance tensile strength, while the cellulose matrix may be creped in order to provide desired properties such as softness and bulk.

Wet strength agents function to provide tensile strength to paper when it is wet, wherein the paper retains more than about 15% of its tensile strength. Tensile strength refers to the ability of the paper product, and its constituent matrices (1) to maintain physical integrity, and (2) to resist tearing, bursting, and shredding under use conditions. In the converting process the parent roll may be cut, embossed, folded and/or rolled in order to prepare the final paper products. The paper industry utilizes wet strength chemistries in the manufacture of items such as tissue paper, paper towels, juice boxes, and milk cartons as well as other cellulosic products. Other industries also utilize wet strength chemistries, such as the textile industry for use as dyeing aids and crease resistance aids; the cosmetics industry in products such as nail polish; and the construction industry in the manufacture of industrial building products such as coatings to prevent the warping of ceiling tiles or as an adhesive for floor tiles. Wet strength agents are generally used in papermaking to enhance the wet strength of the cellulose matrix (or fibrous paper web) via addition to the aqueous slurry.

In U.S. Patent Nos. 2,926,116 and 2,926,154 to Keim et al., cationic thermosetting polyamide-epichlorohydrin resins were used as wet strength agents in papermaking. In U.S. Patent No. 5,993,602 to Smith et al., permanent wet strength agents are used in conjunction with the creping adhesives to prepare creped tissues.

Creping is a process wherein compaction of the cellulose matrix in the machine direction, causes the matrix to become a ridged or microfolded structure. Creping aids have been used in the paper industry for approximately thirty (30) years in order to enhance the natural adhesion of a cellulose matrix or fibrous paper web, constituting the paper at that particular point in the process, to the surface of a drying machine which is used as the final step in the manufacturing process. Strong adhesion between the cellulose matrix and the dryer surface is required to develop a fine crepe structure. Generally, the higher the effective adhesive strength of the creping aid, the softer the resulting paper, however, too great of a level of adhesion may cause a degradation of strength properties.

More particularly, the creping process involves adhering a cellulose matrix to a large, heated, rotating drying or creping cylinder, such as a Yankee dryer, and then removing the adhered cellulose matrix or fibrous paper web with a doctor blade. The doctor blade impacts the creping aid layer just below the cellulose matrix causing the cellulose matrix to buckle and in so doing ruptures some of the interfiber bonds within the cellulose matrix. The severity of this creping action depends upon a number of factors, including the degree of adhesion between the cellulose matrix and the surface of the creping cylinder. In order to increase the adherence of the cellulose matrix to the Yankee dryer, a creping aid is usually sprayed onto the surface of the creping cylinder to supplement any natural adhesion the cellulose matrix may have when applied to the drying cylinder. In order to ensure the creping aid provides a uniform surface on the dryer surface to which the cellulose matrix will be adhered, the solution is passed through an in-line static mixer just prior to the sprayboom.

Attempts have been made to prepare creping aids to improve both paper qualities and machine runnability. Several classes of polymers have been used as creping aids including, but not limited to, thermosetting polymers such as polyamidoamine-epichlorohydrin polymers, polyamine-epichlorohydrin polymer, glyoxylated-polyacrylamide polymers; combinations of polymers such as thermosetting and thermoplastics such as PAE polymers with polyvinyl alcohol (as disclosed in U.S. Patent No. Nos. 4,528,316; 4,501,640; and 4,684,439 to Soerens) and combinations of thermoplastic polymers such as 2-ethyl-2-oxazoline with polyvinylalcohol, ethylene/vinylacetate copolymer or polyvinylpyrrolidone (as disclosed in U.S. Patent No. 4,436,867 to Pomplum) and 2-ethyl-2-oxazoline with polyethyleneimine (as disclosed in U.S. Patent Nos. 5,602,209, 5,980,690 and 5,837,768 to Warchol and Walton); and PAE polymers with phosphates such as monoammonium phosphate and diammoniumphosphate (as disclosed in U.S. Patent No. 4,883,564 to Chen, et. al).

In addition, polyamidoamine-epichlorohydrin resins have been used as creping aids in the following patents: U.S. Patent Nos. 5,388,807 to Espy et al., 5,786,429 to Allen, 5,902,862 to Allen, 5,858,171 and 5,633,309 to Warchol and Walton, and Canadian Patent No. 979,579 to Giles et al. Further, compositions and methods of using polyamine-epihalohydrin resins as a creping aid are disclosed in U.S. Patent No. 5,660,687 to Allen et al. in which a composition comprising polyamine-epihalohydrin resin creping aid and a creping release agent are applied together or separately in the creping process. U.S. Patent No. 5,833,806 to Allen et al. also discloses a method for creping fibrous webs comprising the application of a polyamine-epihalohydrin creping aid.

Polyamine-epihalohydrin resins and their methods of preparation have been described in patents such as U.S. Patent No. 3,248,353 to Coscia and U.S. Patent No. 3,869,342 and 3,869,343 to Munjat. The former discloses the preparation of a cationic, water-soluble polyamine-epichlorohydrin resin polymer. The latter discloses the preparation of cationic water-soluble polyamidoamine-epichlorohydrin polymers.

Other patents which disclose polyamine-epihalohydrin resins include U.S. Patent No. 3,949,014 to Maki et al. in which the polyamine-epichlorohydrin resin is obtained by reacting epichlorohydrin with a polyamine resin having at least two amino groups per molecule, and an amphoteric high molecular weight compound. In addition, U.S. Patent No. 4,129,528 to Petrovich et al. discloses resinous reaction products wherein the hydrohalide salt of a polyamine is condensed with an epihalohydrin to provide a polymer that can improve wet and dry strength when incorporated into cellulosic substrates.

However, while the paper industry has used the polymer compounds previously noted as wet strength agents and creping aids, disadvantages arise in conjunction with their use, particularly the use of epichlorohydrin in the production of resins. Epichlorohydrin, for example, is used in crosslinking polyamidoamines for the production of polyamidoamine-epichlorohydrin resins to act as wet strength agents and creping aids. However epichlorohydrin is a suspected carcinogen, in addition to being flammable and highly toxic. Further, epichlorohydrin reactions can produce byproducts such as dichloropropanols and chloropropanediols which also exhibit an elevated level of toxicity. Polyvinyl alcohol is problematic in that it is expensive to use, resulting in high production costs and it is typically used in conjunction with polyamidoamine-epichlorohydrin chemistries. While it provides adhesion at high temperatures, the use of polyvinyl alcohol results in a hard film, wherein polyamidoamine-epichlorohydrin chemistries are utilized to impart the necessary flexibility to the film. In order to avoid the problems and pitfalls conferred by the use of epichlorohydrin and other organohalides, the use of alternatives, such as polyepoxide crosslinking agents, has been explored in conjunction with the production of resins to be used in the manufacturing of paper.

Embodiments of the present invention address many of the problems and disadvantages currently encountered within the papermaking industry. Several advantages provided by the present invention include allowing for a faster reaction time and lower reaction temperatures (e.g. room temperature versus previous elevated temperatures) resulting in lower production costs, a reduction in production time, and a greater level of safety. Further, embodiments of the present invention are neither suspected carcinogens nor classified as a serious poisons. Still further, the use of an embodiment of the present invention in cross-linking reactions results in commercially viable resins however it does not result in chlorinated byproducts. An embodiment of the present invention is not a flammable material, or a volatile liquid, rather it is a solid material, thereby providing it with safer properties than previous compounds used in cross-linking reactions.

The paper industry's demand for increased paper machine speeds, reduced numbers of process disruptions due to doctor blade changes and paper breaks, softer and more uniform paper, and the need to incorporate the use of safer resin production chemicals necessitates development of new and better creping aids and wet strength agents. Desirable qualities for better creping aids that will improve paper properties and paper machine runnability include: increased adhesion, dispersibility, coating uniformity, and resistance to wet end chemicals. Desirable qualities for wet strength agents include the ability to impart high wet tensile strength/dry tensile strength, resin performance at a level equal to or greater than current industry standards, and low levels or the nonexistence of organochlorides.

### SUMMARY OF THE INVENTION

The present invention relates to resins and processes for preparing and using the same. In particular, resins of the present invention preferably comprise reacting A with K, wherein K is a crosslinking agent, and A is a polyamidoamine. Optionally, a component E may be added to the formula or added separately during the papermaking process.

Preferably, a resin of the present invention has the formula A-K, wherein A is a polyamidoamine, and K is a polyepoxide cross-linking agent selected from one of glycerol triglycidyl ether (triglycidyl glycerol), triphenylolmethane triglycidyl ether, trimethylolethane triglycidyl ether, trimethylolpropane triglycidyl ether, 1,2,4-butanetriol triglycidyl ether, 1,2,6-hexanetriol triglycidyl ether, 1,2,3-heptanetriol triglycidyl ether, pentaerythritol triglycidyl ether, 1,1,1-tris(4-hydroxyphenyl)- ethane triglycidyl ether, calix[4]arene triglycidyl ether, calix[6]arene triglycidyl ether, 4-*t*-butylcalix[4]arene triglycidyl ether, 4-*t*-butylcalix[6]arene triglycidyl ether, pyrogallol triglycidyl ether, 1,2,4-benzenetriol triglycidyl ether, phloroglucinol triglycidyl ether, and triglycidylisocyanurate. More preferably, K is a polyepoxide cross-linking agent selected from one of glycerol triglycidyl ether (triglycidyl glycerol), trimethylolethane triglycidyl ether, trimethylolpropane triglycidyl ether and 1,3,5-triglycidylisocyanurate (TGIC). Most preferably, however, the cross-linking agent K is 1,3,5-triglycidylisocyanurate (TGIC), also known as triglycidylisocyanurate or Tris(2,3-epoxypropyl)isocyanurate (TEPIC).

The present invention is also directed to a process for the preparation of resins comprising the formula A-K, which comprises reacting A with K, wherein K is a cross-linking agent, and A is a polyamidoamine.

The solution of A, preferably has from 30% to 70% by weight based on solids, more preferably from 40% to 65% by weight based on solids, and most preferably 50% by weight based on solids.

The present invention is further directed to a resin having the formula A-K, in a solution having a solids content from 10 to 50% by weight based on solids, preferably from 15% to 45% by weight based on solids.

When used as a creping aid, a resin of the present invention may be applied to a surface, typically the surface of a dryer, in a form comprising aqueous, dispersion, or aerosol. A release agent may also be applied to the drying surface. The creping aid together with the release agent may be simultaneously or separately applied to the surface. The release agent may be applied to the dryer and/or forming fabric. The release agent may be applied to the drying surface in combination with the creping aid using the same sprayboom or using different spray booms. Application may be through a single sprayboom at a point just prior to the transfer of the wet cellulose matrix to the dryer surface.

Resins of the present invention can be used as wet strength agents, and/or creping aids for preparing cellulosic products, dye fixatives for preparing textiles or cellulosic products, incorporated into card stock for preparing shingles or roof tops, as an additive for preparing cosmetics and hair conditioners, as a crease resistance aid for textiles, as an adhesive for floor tiles or as a backing agent for ceiling tiles.

A resin of the present invention can also be used to prepare cellulosic products, wherein the amount of the resin is from 0.005 to 2.5 dry kg/dry t (0.01 to 50 dry lbs/dry ton) of total weight of the cellulosic products.

The present invention is also directed to cellulosic products prepared by the process which comprises adding at least one wet strength agent to a cellulosic slurry and/or adding at least one creping aid to a drying surface.

In addition, the present invention relates to cellulosic products containing the resins described above.

### DETAILED DESCRIPTION OF THE INVENTION

In the context of this disclosure and in accordance with the present invention, a number of terms shall be utilized.

The term "cellulosic products" refers to paper products containing cellulose including but not limited to paperboards, writing papers, facial tissues, bathroom tissue, paper towels and napkins.

The term "wet strength" refers to the ability of the paper to retain more than 15% of its tensile strength when wetted out after immersion in water.

The term "wet strength resin" refers to a polymer that when incorporated into a cellulosic product will impart wet strength properties to the cellulosic product.

The term "cellulosic fiber web" refers to the wet paper web made by a process which includes forming a papermaking furnish; depositing the furnish onto a surface, removing water from the web; and adhering the sheet to a drying surface such as a Yankee Dryer, or alternatively a through-air dryer or dryers preceding the Yankee dryer or may have no Yankee dryer or more than one Yankee dryer, and removing the sheet by a creping blade such as a doctor blade.

One aspect of the present invention is directed to a resin comprising the formula: A-K, which is a reaction product of A and K, resulting in a resin, C, wherein A is a creping precursor comprising at least one cross-linkable functional group; and K is a cross-linking agent. The mole ratio of the reactive functionalities of K:A is important in determining the properties of the final polymer. When reacting A with K, the mole ratio of reactive functionalities of K to A is at least about 1:1. The mole ratio of the reactive functionalities of K:A can be varied to suit particular utilities. That is, the mole ratio of the reactive functionalities of K:A depends on the properties the user wants the final resin to impart into the paper. For example, if more wet strength properties are desired in the paper resulting from the use of the resin, then the mole ratio of the reactive functionalities of K:A needs to be high in order to have enough functionality to impart wet strength properties to the resulting paper. However, if creping aid properties, such as greater flexibility, are desired in the paper, then the mole ratio of the reactive functionalities of K:A must be adjusted to result in improved paper flexibility while retaining solubility in water.

A is a water-soluble and/or dispersible polyamidoamine. Examples of A of the present invention include, but are not limited to, any known precursor having at least one cross-linkable functional group known in the art, for example the polymers recited in U.S. Patent Nos. 3,869,342 and 3,869,343 to Munjat et al., wherein such polymers are the reaction products of a mixture of (a) itaconic acid with (b) an amino acid and/or a lactam and (c) a diamine and/or a polyalkylene-polyamine.

Suitable polyamidoamines preferably include, but are not limited to, precursors, including end-capped polyamidoamines as covered in U.S. Patent 5,786,429 and U.S. Patent 5,902,862, and at least one of a polyamidoamine derived from adipic acid-diethylenetriamine, dimethylglutarate-diethylenetriamine, caprolactam-itaconic acid-diethylenetriamine, caprolactam-itaconic acid-6-aminohexanoic acid-diethylenetriamine, methylbisamino propylamine-oxalicacid-urea.

In a general and representative sense, to prepare a polyamidoamine from a diacid and a polyalkylene-polyamine; and optionally containing in addition, an amino acid, lactam and/or diamine; a mixture of the reactants is preferably heated at a temperature of about 125-200°C for preferably about 0.5 to 4 hours, at atmospheric pressure. Where a reduced pressure is employed, lower temperatures such as about 75°C to about 150°C may be utilized. This polycondensation reaction produces water as a byproduct, which is removed by distillation. At the end of this reaction, the resulting product is typically dissolved in water at a concentration of about 50% by weight total polymer solids. Where a diester is used instead of a diacid, the prepolymerization can be conducted at a lower temperature, preferably about 100-175°C at atmospheric pressure. In this case, the byproduct will be an alcohol, the type of alcohol depends upon the identity of the diester. For example, where a dimethyl ester is employed the alcohol byproduct will be methanol, while ethanol will be the byproduct obtained from a diethyl ester. Where a reduced pressure is employed, lower temperatures such as about 75°C to about 150°C may be utilized.

Examples of the at least one cross-linkable functional group of A preferably includes, but is not limited to, epoxides, azetidiniums, carboxylic acids, phosphonic acids, phosphoric acids, sulfuric acids, sulphonic acids, esters, alkyl halides, aromatic halides, alcohols, phosphates, sulfonates, anhydrides, amines such as primary, secondary and tertiary amines, alkeneimine, more preferably, at least one of carboxylic acids, esters, epoxides, azetidiniums, and amines such as primary, secondary and tertiary amines, and most preferably, at least one of amines such as primary, secondary and tertiary amines.

In an embodiment of the present invention, A is a reaction product of at least one polyfunctional compound including, but not limited to, polyfunctional acids, polyfunctional esters, polyfunctional aminoacids, polyfunctional amines and polyfunctional anhydrides. Preferably, A is a reaction product of at least one polyfunctional acid, polyfunctional aminoacid, polyfunctional ester, polyfunctional anhydride with at least one polyfunctional amine and combinations thereof. More preferably A is a reaction product of polyfunctional acids or polyfunctional esters with a polyfunctional amine. Most preferably A is a polyamidoamine or polyamine.

Suitable polyfunctional acids preferably include, but are not limited to, glutaric (GLU), adipic (AD), itaconic (IT), azeleic, sebacic, succinic oxalic and combinations thereof, more preferably itaconic (IT), adipic (AD), glutaric (GLU) and combinations thereof, and most preferably, adipic (AD) and glutaric (GLU) and combinations thereof.

Suitable polyfunctional esters preferably include, but are not limited to, dimethylglutarate (DMG), dimethylsuccinate, dimethyladipate (DMA) and combinations thereof, and more preferably dimethylglutarate (DMG), dimethyladipate (DMA) and combinations thereof.

Suitable polyfunctional aminoacids preferably include, but are not limited to, caprolactam, 6-aminohexanoic acid, polylysine, polyalanine, polyhistidine, peptides and combinations thereof, more preferably caprolactam, polylysine, polyhistidine and combinations thereof, and most preferably caprolactam, polylysine and combinations thereof. Suitable peptides preferably include, but are not limited to, natural polyaminoacids or polyaminoacids synthesized from natural or commercially available aminoacids or modified aminoacids. More preferably, the peptides comprise at least one lysine.

Suitable polyfunctional amines preferably include, but are not limited to, ethylenediamine (ED), diethylenetriamine (DETA), triethylenetetramine (TETA), tetraethylenepentamine (TEPA) and higher homologues, hexamethylenediamine (HMD), bis(hexamethylene)triamine (BHMT) and higher homologues, poly(oxypropylene)diamine, poly(oxyethylene)diamine, N-(2-aminoethyl)-1,2-ethanediamine, N,N'-1,2-ethanediylbis(1,3-propanediamine), N-methyl-bis(3-aminopropyl)amine (MBAPA), polyvinyl amine, 1,3-diaminopentane, urea, spermine, spermidine, propylenediamine (PD), dipropylenetriamine (DPTA), tripropylenetetramine (TPTA), tetrapropylenepentamine (TPPA) and higher homologues, hexapropylenediamine (HPD), bis(hexapropylene)triamine (BHPT) and higher homologues, polymethyldiallylamine and combinations thereof, more preferably diethylenetriamine (DETA), triethylenetetramine (TETA), tetraethylenepentamine (TEPA), hexamethylenediamine (HMD), and N-methyl-bis(3-aminopropyl)amine (MBAPA), dipropylenetriamine (DPTA), and tripropylenetetramine (TPTA).

Suitable polyfunctional anhydrides preferably include, but are not limited to adipic anhydride, glutaric anhydride, itaconic anhydride, sebacic anhydride, azeleic anhydride and combinations thereof.

A, of the present invention, may be prepared by any method known in the art, such as reacting polyfunctional compounds. For example, the process of preparing a polyamidoamine version of polymer A includes a condensation type reaction between either a polyfunctional acid, polyfunctional ester, or polyfunctional anhydride and a polyfunctional amine. In the condensation reaction, the reactants are mixed together and then heated until the amide bonds are formed and the polymer reaches the desired molecular weight, which in this instance was monitored via viscosity, estimated using In-process Procedure 2 by comparing Gardner-Holdt bubble tube viscosity measurement made on a 50% solution of polyamidoamine polymer at 25°C with Gardner-Holdt standards.

The reaction also typically produces a byproduct such as water for the reaction of a polyfuncional acid and polyfunctional amine or methanol in the case of a polyfunctional methylester and polyfunctional amine. The byproduct is generally distilled away from the growing polymer. In the example of the polyfunctional acid and polyfunctional amine reaction, once the polymer has reached the desired viscosity, water may be added to the polymer solution to reduce the viscosity to a more desirable viscosity for subsequent reactions.

In a general and representative sense, the method of the present invention for the preparation or synthesis of a precursor A from itaconic acid, caprolactam and diethylenetriamine (DETA) can be carried out as follows: 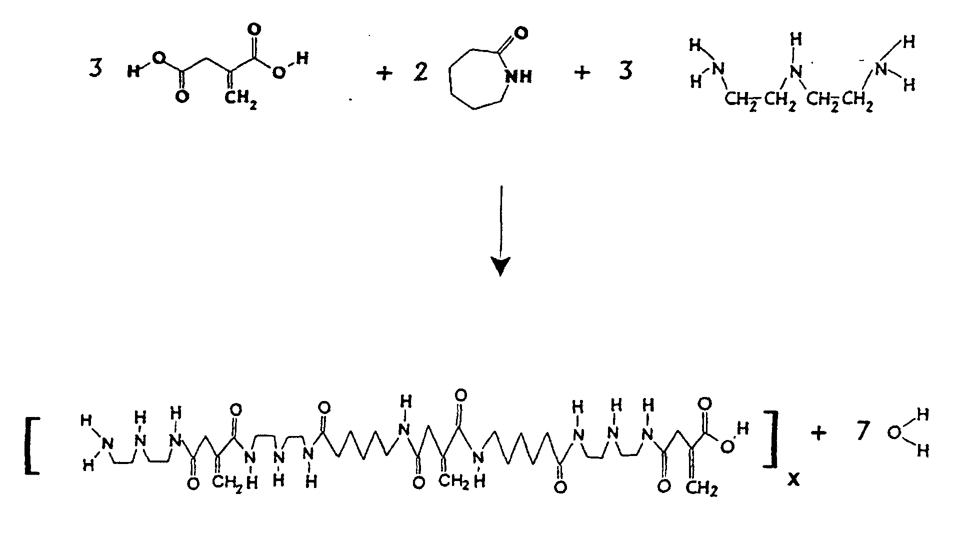 where "x" is the number of sub-units that make up the polymer as described above.

A solution of A, preferably has from 30% to 70% by weight based on solids, more preferably from 40% to 65% by weight based on solids, and most preferably 50% by weight based on solids.

Another aspect of the present invention is directed to a cross-linking agent, K. The cross-linking agent is a polyepoxide selected from one of glycerol triglycidyl ether (triglycidyl glycerol), triphenylolmethane triglycidyl ether, trimethylolethane triglycidyl ether, trimethylolpropane triglycidyl ether, 1,2,4-butanetriol triglycidyl ether, 1,2,6-hexanetriol triglycidyl ether, 1,2,3-heptanetriol triglycidyl ether, pentaerythritol triglycidyl ether, 1,1,1-tris(4-hydroxyphenyl)- ethane triglycidyl ether, calix[4]arene triglycidyl ether, calix[6]arene triglycidyl ether, 4-*t*-butylcalix[4]arene triglycidyl ether, 4-*t*-butylcalix[6]arene triglycidyl ether, pyrogallol triglycidyl ether, 1,2,4-benzenetriol triglycidyl ether, phloroglucinol triglycidyl ether, and triglycidylisocyanurate. More preferably, K is a polyepoxide cross-linking agent selected from one of glycerol triglycidyl ether (triglycidyl glycerol), trimethylolethane triglycidyl ether, trimethylolpropane triglycidyl ether and 1,3,5-triglycidylisocyanurate (TGIC). Most preferably, the cross-linking agent K is 1,3,5-triglycidylisocyanurate (TGIC), also known as triglycidylisocyanurate or Tris(2,3-epoxypropyl)isocyanurate (TEPIC).

In a general and representative sense, the method of the present invention for the preparation or synthesis of a resin, C, of the present invention, comprising the formula A-K, for use as a creping aid and/or wet strength agent, can be carried out as follows:

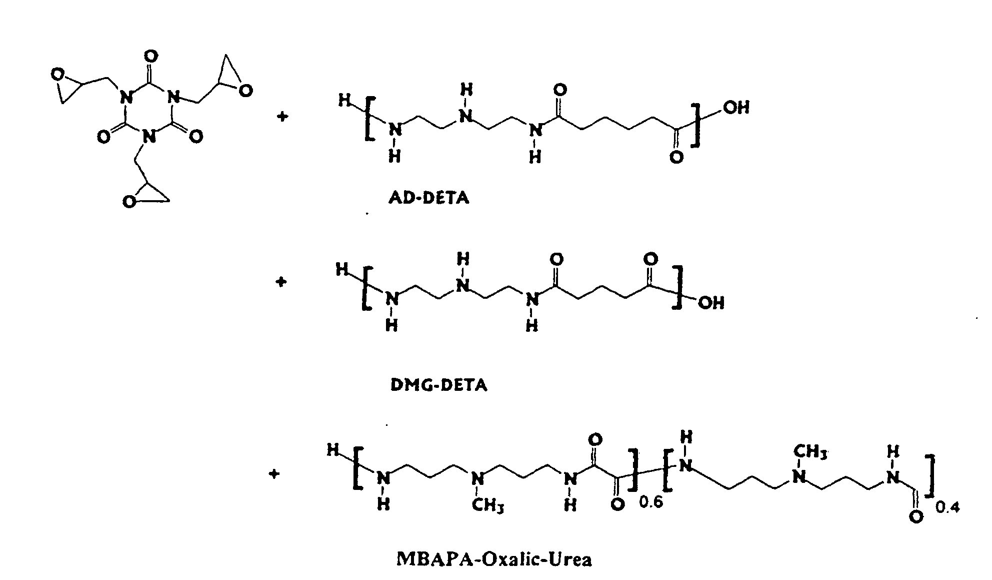

The reaction of A and K was typically performed at about 10 to 60% total solids (A + K) in water. More preferably the reaction is carried out at a solids level of 20 to 40% and most preferably the reaction is carried out at a solids content of 25 to 35%. The prepolymer (A) and crosslinker (K) were mixed with the appropriate amount of dilution water and the mixture was then usually heated. The temperature of the reaction can range from 20°C to 100°C, more preferably in the range of 30 to 80°C and most preferably in the range of 50 to 70°C. The appropriate combination of reaction solids, reaction temperature and ratio of K to A were chosen such that the reaction proceeded at a reasonably fast rate to ensure efficient production while avoiding an excessively fast rate of reaction that could lead to gelation and loss of control over the desired product properties. The viscosity of the reaction mixture was monitored with time. Typically this involved the use of Gardner-Holt viscosity tubes. When the reaction mixture reached the appropriate viscosity level on the Gardner-Holt scale dilution water and/or a stabilizing acid were added to end the reaction. Alternately, the reaction may be diluted with warm water and the heating continued until the viscosity again builds to the desired level. Several such iterations can be performed before ending the reaction by the addition of dilution water and/or stabilizing acid.

When a reaction of A and K, is quenched with an acid, the resin solution preferably is 15 to 50 % by weight based on solids, more preferably 20 to 45% by weight based on solids, and most preferably 15 to 25% by weight based on solids.

When the reaction of A and K is quenched with a sulfite, the resin preferably is 10 to 60 % by weight based on solids, more preferably 15 to 45 % by weight based on solids, and most preferably up to 44% by weight based on solids.

Examples of the resin produced according to a method of the present invention include, but are not limited to, the following:
1. A is adipic acid-diethylenetriamine polymer; and K is trigylcidylisocyanurate.
2. A is caprolactam-itatonic acid-diethylenetriamine polymer; and K is trigylcidylisocyanurate.
3. A is caprolactam-itatonic acid-6 aminohexanoic acid-diethylenetriamine polymer; and K is trigylcidylisocyanurate.
4. A is dimethylglutarate-diethylenetriamine polymer; and K is trigylcidylisocyanurate.
5. A is methylbisaminopropylamine-oxalic acid -urea polymer; and K is trigylcidylisocyanurate.
6. A is adipic acid-diethylenetriamine polymer; and K is glycerol triglycidyl ether (triglycidyl glycerol).
7. A is caprolactam-itatonic acid-diethylenetriamine polymer; and K is glycerol triglycidyl ether (triglycidyl glycerol).
8. A is caprolactam-itatonic acid-6 aminohexanoic acid-diethylenetriamine polymer; and K is glycerol triglycidyl ether (triglycidyl glycerol).
9. A is dimethylglutarate-diethylenetriamine polymer; and K is glycerol triglycidyl ether (triglycidyl glycerol).
10. A is methylbisaminopropylamine-oxalic acid -urea polymer; and K is glycerol triglycidyl ether (triglycidyl glycerol).
11. A is adipic acid-diethylenetriamine polymer; and K is trimethylolethane triglycidyl ether.
12. A is caprolactam-itatonic acid-diethylenetriamine polymer; and K is trimethylolethane triglycidyl ether.
13. A is caprolactam-itatonic acid-6 aminohexanoic acid-diethylenetriamine polymer; and K is trimethylolethane triglycidyl ether.
14. A is dimethylglutarate-diethylenetriamine polymer; and K is trimethylolethane triglycidyl ether.
15. A is methylbisaminopropylamine-oxalic acid -urea polymer; and K is trimethylolethane triglycidyl ether.
16. A is adipic acid-diethylenetriamine polymer; and K is trimethylolpropane triglycidyl ether.
17. A is caprolactam-itatonic acid-diethylenetriamine polymer; and K is trimethylolpropane triglycidyl ether.
18. A is caprolactam-itatonic acid-6 aminohexanoic acid-diethylenetriamine polymer; and K is trimethylolpropane triglycidyl ether.
19. A is dimethylglutarate-diethylenetriamine polymer; and K is trimethylolpropane triglycidyl ether.
20. A is methylbisaminopropylamine-oxalic acid -urea polymer; and K is trimethylolpropane triglycidyl ether.

In addition, the process for preparing a resin of the present invention can further include the addition of at least one optional component E or a combination thereof. Suitable optional components preferably include, but are not limited to defoamers, preservatives, and corrosion inhibitors. Defoamers can also be used to reduce the amount of foam produced during manufacturing. Examples of defoamers preferably include, but are not limited to ethoxylated amine, Jeffamine® manufactured by Huntsman and Advantage® 831 manufactured by Hercules Incorporated.

Preservatives can be also be used to preserve a resulting resin from microbial growth. Examples of preservatives preferably include, but are not limited to, Kathon® manufactured by Rohm & Haas, PABA, Proxcel® manufactured by Zeneca, and potassium sorbate.

Corrosion inhibitors can also be added so that when the resulting resin is applied on a metal surface, corrosion is prevented. Suitable corrosion inhibitors preferably include, but are not limited to, triazines, dibasic acid salts, nitrites, and Hostacor® manufactured by Hoechst-Celanese.

Alternatively, a resin of the present invention can be applied onto the surface of the Yankee dryer, e.g., by spraying, thereby acting as a creping aid. After this creping aid is applied to the Yankee dryer, the cellulosic fiber web is pressed onto the Yankee dryer. Thus, the creping aid is indirectly added to the cellulosic fiber web.

The production of a cellulosic fiber web includes forming a papermaking furnish; depositing the furnish onto a foraminous surface, removing water from the web; and adhering the sheet to a drying surface such as a Yankee Dryer, and removing the sheet by a creping blade such as a doctor blade. Alternatively, the process may include a through-air dryer or dryers preceding the Yankee dryer or may have no Yankee dryer or more than one Yankee dryer. A wet strength agent produced by a method of the present invention can be applied onto the surface of the Yankee dryer alone or simultaneously with a creping aid formulation. Alternatively, the wet strength agent can be included with a creping aid formulation and then applied to the cellulosic fiber web.

When the resin solution of the present invention, having the formula A-K, is used as a wet strength agent, the resin preferably has from 10% to 50% by weight based on solids; more preferably from 10% to 45% by weight based on solids; and most preferably from 15% to 45% by weight based on solids.

A resin of the present invention prepared according to a method of the present invention can be used in a process for preparing cellulosic products. Specifically, a resin can be used as a wet strength resin and/or a creping aid to prepare cellulosic products. The cellulosic products of the present invention can contain the resin, C, preferably in an amount from 0.005 kg/dry t (0.01 lbs./dry ton) to 25 dry kg/dry t (50 dry lbs./dry ton) of total weight, more preferably from 0.05 kg/dry t (0.1 lbs./dry ton) to 15 dry kg/dry t (30 dry lbs./dry ton) of total weight. A wet strength resin of the present invention can be added to a paper furnish (cellulosic slurry) in an amount from 0.005 kg/t (0.01 lbs./ton) to 25 kg/t (50 lbs./ton), preferably from 5 kg/t (10 lbs./ton) to 15 kg/t (30 lbs./ton), and more preferably from 7.5 kg/t (15 lbs./ton) to 15 kg/t (30 lbs./ton) depending on the grade of cellulosic product being manufactured. For instance, a tissue product may need little or no wet strength agent; whereas a towel may need higher amounts of a wet strength agent.

Further, a resin of the present invention can also be used as a creping aid to prepare cellulosic products. More specifically, the creping aid can be used in a creping process to prepare cellulosic products.

The creping process of the present invention can include the steps of applying the creping aid to a drying surface, preferably a surface of a Yankee Dryer, to provide an adhesive coating, adhering the cellulosic fiber web to the drying surface by pressing the cellulosic fiber web against the adhesive coating surface, and creping the cellulosic fiber web with a creping device to dislodge it from the drying surface. Alternatively, a creping aid of the present invention can be applied to the cellulosic fiber web prior to the web being applied to the Yankee Dryer to create the requisite adhesion to the drying surface.

The application of a creping aid of the present invention can be done by any technique known in the art, and in the form of aqueous, dispersion or aerosol. Preferably, the creping aid is applied via a spray boom directed at the surface of the drying surface prior to applying the cellulosic fiber web onto the surface. Spray application of the creping aid can be performed according to any known method in the art.

A creping aid of the present invention may be applied onto a drying surface in a form comprising aqueous, dispersion, or aerosol to a surface. A polyvinyl alcohol and/or release agent may also be applied onto the drying surface. The creping aid together with the polyvinyl alcohol and/or release agent may be simultaneously or separately applied onto the surface. The polyvinyl alcohol may be applied to the dryer. The release agent may be applied to the dryer and/or forming fabric.

When the resin, C, of the present invention, having the formula A-K is used as a creping aid, the resulting cellulosic product of the present invention preferably contains from 0.005 kg/dry t (0.01 lbs./dry ton) paper to 2.5 kg/dry t (5 lbs./dry ton) paper; more preferably from 0.005 kg/dry t (0.01 lbs./dry ton) paper to 1.5 dry kg/dry t (3 dry lbs./dry ton) paper, and most preferably from 0.05 kg/dry t (0.1 lbs./dry ton) paper to 0.025 dry kg/dry t (0.5 dry lbs./dry ton) paper.

In addition, a creping aid of the present invention can be applied onto the surface of the Yankee dryer alone or simultaneously with a formulation containing at least one release agent.

Alternatively, a creping aid of the present invention can be included in a formulation containing at least one release agent prior to being applied to the cellulosic fiber web. Release agents aid in the uniform release of the tissue web at the creping blade and lubricate as well as protect the blade from excessive wear when the tissue web is being creped from the Yankee Dryer.

Suitable release agents include, but are not limited to, (1) mineral oil-based such as emulsifiable oil and may contain an imidazoline quat; (2) vegetable oil-based such as emulsifiable vegetable oil; (3) synthetic oil such as polyethylene glycols, polypropylene glycols and mono or diesters thereof; and (4) water-based release agents such as water-soluble soap.

When a mineral oil-based release agent is used, it is generally used in conjunction with either a static mixer or an emulsion unit to ensure uniform application.

When a water-soluble soap is used, it is generally used in conjunction with a preservative and defoamer to reduce foaming.

A creping aid package may be applied in conjunction with polyvinyl alcohol. The amount of the creping aid of the present invention and the release agent used in a creping process is the effective amount of the creping aid that adheres the cellulosic fiber web to the drying surface and the effective amount of the release agent that releases the cellulosic fiber web from the drying surface.

The use of a resin of the present invention as a creping aid to prepare cellulosic products imparts excellent paper qualities to the resulting paper and at the same time enhance paper machine runnability which includes increased adhesion, dispersibility, coating uniformity, and resistance to wet end chemicals.

An embodiment of the present invention can also be used in various other industries as a wet strength agent. Typically, the textile industry uses wet strength agents as a dye fixative or to improve crease resistance. Additionally, the resins of the present invention can be used to manufacture card stock for preparing roof tops or making shingles. It is incorporated in the manufacture of card stock to reduce stiffness, and thus improve machine runnability while not changing the absorbency properties of the card stock, and thus does not interfere with coating ability of roofing tar and asphalt. Further the resins of the present invention may be used as adhesives for floor tiles and as backing agents for ceiling tiles. The resins of the present invention can also be used as an additive in cosmetics and personal care products, such as hair conditioners and nail polish.

The embodiments of the present invention are further defined in the following Examples. It should be understood that these Examples, while indicating preferred embodiments and the most preferred embodiments of the present invention, are given by way of illustration only. From the above discussion and these Examples, one skilled in the art can ascertain the essential characteristics of this invention, and without departing from the scope thereof, can make various changes and modifications of the invention to adapt it to various uses and conditions. Thus various modifications of the present invention in addition to those shown and described herein will be apparent to those skilled in the art from the foregoing description. Although the invention has been described with reference to particular means, materials and embodiments, it is to be understood that the invention is not limited to the particulars disclosed, and extends to all equivalents within the scope of the claims.

### EXAMPLES

### I. Preparation of the Polyamidoamine Pre-polymers

Examples of the synthesis of three types of polyamidoamine prepolymer are given below. Three common polyamidoamines are (1) reaction product of a polyacid and polyamine; (2) reaction product of an amino acid with itself; with a polyamine or with a polyamine and polyacid; and (3) reaction product of a diester and polyalkylenepolyamine.

### A. Polyacid reacting with a polyamine

A typical example is the reaction of adipic acid (AD) with diethylenetriamine (DETA). DETA (24.8%) and 8.3% soft water, blended in a reaction vessel. Adipic (33.5%) acid is slowly added. A strong exothermic reaction takes place. The temperature of the reaction vessel should be controlled to keep the reaction below 115°C. Once the adipic acid has been completely added, the batch is heated to about 160-168°C in order to polymerize the polyacid and polyamine forming the polyamidoamine prepolymer (AD-DETA) and to distill the water byproduct. When the polyamidoamine reaction reaches the appropriate viscosity, measured via Gardner-Holdt In-process Procedure 1, the remaining 33.5% water is added to the batch and the reactor is allowed to cool to room temperature. This reaction produces a 50% solids solution of AD-DETA prepolymer.

### B. Polyacid reacting with aminoacid and polyamine stepwise

Suitable prepolymers of this type are covered under U.S. Patents 3, 869,342 and 3, 869,343, and are incorporated herein.

Pre-polymer of caprolactam, itaconic acid, diethylenetriamine may be prepared stepwise. Caprolactam (13.85%) or a combination of caprolactam and 6-aminohexanoic acid and 2.20% water is added to the reaction vessel. Itaconic acid (7.96%) is added to the reactor. The reaction is stirred and heated to about 118 - 121°C. The reaction is refluxed at that temperature for about 2 hours and then cooled to about 66°C. Slowly, 12.62% diethylenetriamine is added. The resulting exotherm is controlled so that the temperature does not exceed 110°C in order to prevent loss of DETA. The reaction is heated to reflux at about 127-132°C for two hours. The reaction is cooled to about 76°C and a second portion of itaconic acid (15.92%) is added. The reaction is heated to reflux at about 127-132°C for two hours. The reaction is cooled to about 93°C and then slowly the second portion of diethylenetriamine (6.31 %) is added. The exotherm is controlled to below 116°C. The reaction is heated to about 166°C until the final viscosity specification as measured using Gardner-Holdt In-process Procedure 1 is met. The final portion of water (41.13%) is added and the reaction is allowed to cool to room temperature. The reaction produces the polyamidoamine as a 50% solids solution.

### Gardner-Holdt In-process Procedure 1:

1. The hot molten polymer should be thinly spread on the clean metal plate. The metal plate should be small enough to be placed into the refrigerator.
2. Place the polymer covered metal plate into the refrigerator for 3-10 minutes until the solid is brittle.
3. Pulverize a sufficient amount of the solid polymer (about 30 g) in a mortar and pestle.
4. Weigh 25g of the pulverized polymer in to a 250 mL beaker equipped with a stirrer.
5. To prepare the 50% polymer solution, add 25g of warm or hot water to the beaker to dissolve the pulverized polymer.
6. Place the beaker onto a hot plate and warm to further dissolve the polymer.
7. When the polymer is completely dissolved, reweigh the 50% solution and replenish any water that has evaporated during the heating period.
8. Transfer the 50% polymer solution into an empty Gardner-Holdt tube up to the fill line, seal with a cork and cool the solution under tap water to 25°C.
9. Compare the rate at which the bubble rises in the polymer solution tube to the Gardner-Holdt viscosity standard tubes
10. The reaction should be run until the polymer (50% solution) has a Gardner-Holdt viscosity between T-U at 25°C.

### C. Diester reacting with polyalkylene-polyamine

Dimethylester is reacted with the polyamine on a one to one mole basis. Both reactants are placed into the reaction vessel at room temperature. The vessel is heated to about 104°C and the temperature is slowly raised to about 110°C or until the first sign of methanol distillation begins. Once the bulk of the methanol is distilled over, the reaction temperature is raised to about 110°C to complete the reaction. With the vessel in a reflux mode, water is added to yield a solution of up to 65% solids.

### II. Preparation of a CAP-IT-DETA Pre-polymer

Precursor A was prepared by blending about 13.85 wt.% caprolactam or a combination of caprolactam and 6-aminohexanoic acid and about 2.2 wt.% water in a reaction vessel. 7.96 wt.% itaconic acid was slowly added to the vessel producing a strong exothermic reaction. The reaction was stirred and heated to about 118-121°C. The reaction was refluxed for about two hours at that temperature and then cooled to about 66°C. 12.62 % diethylenetriamine was slowly added to the reaction vessel. The exothermic reaction was maintained by controlling the temperature at a maximum temperature of about 110°C. The reaction was heated to reflux at about 127-132°C for two hours. The reaction was cooled to about 77°C and a second portion of the itaconic acid (15.92 wt.%) was added. The reaction was then heated to reflux at about 127-132°C for about two hours. The reaction was cooled to about 93°C and the second portion of diethylenetriamine (6.31wt. %) was slowly added. The exothermic reaction was controlled by maintaining the reaction temperature below about 116°C. The reaction was heated to about 166°C for about 14-16 hours without vacuum. At the end of that time, a vacuum was slowly applied to assist in water removal. The reaction was kept at this temperature under vacuum until the final viscosity specification was met. The final portion of water (41.13 wt. %) was added and the reaction was allowed to cool. The reaction produced a 50% solids solution of polyamidoamine polymer.

### III. Preparation of an AD-DETA-TGIC Resin

The present invention also contemplates a process for preparing a resin, having the formula A-K, comprising about a 16:1 mole ratio of Adipic acid -diethylenetriamine pre-polymer (AD-DETA) to triglycidylisocyanurate crosslinking agent (TGIC). About 333.3 g of distilled water and about 160.0g (0.376 moles) of a 50% solids solution of AD-DETA pre-polymer (having a repeat unit molecular weight of 213g/mole), prepared as described in Example 1, were added to a clean round bottom flask equipped with a stirrer, heating mantle and temperature monitoring device. The reaction was heated to about 30-35°C and agitation of the flask was continued throughout the reaction. Subsequently, about 6.7g (0.023 moles) of TGIC (having a molecular weight of 297g/mole) was added to the round bottom flask. The viscosity of the reaction was monitored via Gardner-Holdt Viscosity bubble tubes as per In-process Procedure 2 provided below. Once the viscosity has reached a Gardner-Holdt viscosity of "F", 200g of distilled water was added to the round bottom flask. An amount of sulfuric acid was then added until the pH of the reaction was adjusted to 3 to 3.5.

The viscosity of the above described reaction was monitored using Gardner-Holdt viscosity bubble tubes and was conducted according to In-process Procedure 2 described as follows: before starting the reaction, the Gardner-Holdt viscosity tubes A to K were warmed to about 30-35°C in a constant temperature bath. Once the reaction solution has reached about 30-35°C, the viscosity increase was monitored. Approximately10 ml sample of the reaction solution was withdrawn and placed into an empty Gardner-Holdt tube, thereafter the tube was sealed with a cork. The appropriate Gardener-Holdt standard tubes and the sample tube were placed into a tube holder. The holder was inverted and the relative times for the air bubbles in each tube to go from the bottom to the top of the tube were compared. The sample was then returned to the reaction flask. The viscosity was checked at ten-minute intervals until the viscosity of the batch reached Level "A". Once the viscosity of the batch reached Level "A", the viscosity of the growing resin was checked at five-minute intervals until the resin solution reached a viscosity of Level "B". Once the batch reached a viscosity of Level "B", the resin solution viscosity was checked at two-minute intervals until the resin solution reached the desired termination viscosity of "F".

### IV. Preparation of DMG-DETA-TGIC Resin

The present invention is further directed to a process for preparing a resin, C, having the formula A-K, comprising about a 1:1 mole ratio of Dimethylglutarate-diethylenetriamine pre-polymer (DMG-DETA) to triglycidylisocyanurate crosslinking agent (TGIC) which may be prepared as follows: about 300.0g of distilled water and about 31g (0.104 moles) of TGIC (having a repeat unit molecular weight of 297g/mole) were added to a clean 1-liter round bottom flask equipped with a stirrer, a heating mantle and a temperature monitoring device. The reaction was heated to about 70-77°C. Agitation was started and continued until the reaction was complete. Once the TGIC went into solution and became a clear, colorless liquid, the reaction was held at this temperature for about 30 minutes. After about 30 minutes, the reaction was allowed to cool to below about 45°C. Subsequently, about 40.0g (0.101 moles) of a 50% solids solution of Dimethylglutarate-DETA pre-polymer (having a molecular weight of 199g/mole) was added to the reaction vessel. The pH was adjusted to about 9 and the temperature was maintained between about 30-35°C. The viscosity increase of the reaction solution was monitored via Gardner-Holdt Viscosity bubble tubes as per In-process Procedure 2 as provided above. Once the viscosity had reached a Gardner-Holdt viscosity of "D", about 300g of distilled water was added. An amount of sulfuric acid was then added until the reaction pH was adjusted to between 3 and 3.5.

When a reaction of A and K, is quenched with an acid, the resin solution preferably is about 15 to 50 % by weight based on solids, more preferably about 20 to 45% by weight based on solids, and most preferably about 15 to 25% by weight based on solids.

When the reaction of A and K is quenched with a sulfite, the resin preferably is about 10 to 60 % by weight based on solids, more preferably about 15 to 45 % by weight based on solids, and most preferably up to about 44% by weight based on solids.

### V. Adhesive Strength Test

### A. Evaluation of TGIC Resin Adhesive Strength

The TGIC resins were evaluated for adhesive strength. The TGIC resins have adhesive strength similar to their epichlorohydrin analogs. The Adipic acid-DETA-TGIC (AD-DETA-TGIC) and Dimethylglutarate-DETA-TGIC (DMG-DETA-TGIC) resins were also prepared and evaluated wherein all have sufficient adhesive strength to be used as creping aids. The creping aid chemistries included Rezosol® 8223, a polyamidoamine-epichlorohydrin resin made available by Hercules Incorporated as well as Unisoft 805® and Unisoft 805A®, also polyamidoamine-epichlorohydrin resins made available by Hercules Incorporated.

Adhesive strength of the creping aids was determined using the Tinius-Olsen Testing Machine Model 5000 (manufacture by Tinius Olsen Testing Machine Co., Willow Grove, PA) by a 180° Peel Test Method, which is a modification of the ASTM Method D903 ("Standard Test Method for Peel or Stripping Strength of Adhesive Bonds") as described hereunder.

The procedures for measuring the adhesive strength of the are as follows:

### Sample Preparation

1. Steel plates (Catalog No. 101-10-10, SAE 1010 cold rolled heavy gauge steel plates, Metaspec Co.; San Antonio, TX) are submerged in a petroleum ether bath for at least 30 minutes to remove the rust preventive coating. The plates are then washed with a surfactant (Cerfak® 1400 manufactured by Houghton International), rinsed with acetone, and then air-dried.
2. 1" X 8" non-woven fabric strips (30% natural cellulose/70% polyester, Staple Sewing Aids Corp., NJ) are cut from the material such that all strips are cut in the same direction and so that the same side of the fabric adheres to the metal plate.
3. The fabric strips are placed in 3% solids solutions of the creping aid and thoroughly soaked for at least 15 minutes. A minimum of two strips are run per sample. After soaking, the fabric strips are removed from the solution and excess solution is allowed to drip off. Next the strips are placed onto the metal surface so that one end of the fabric strip is flush with the edge of the metal plate. The strip is centered on the metal panel. The fabric strip is smoothed onto the metal surface such that no air bubbles form under the fabric strip. Smoothing is carefully done to avoid loss of adhesive solution.
4. The fabric coated plate is then placed onto a preheated (15 minutes minimum) Corning Hot Plate Stirrer (model # PC351) on setting 2 (about 250°C) for four minutes. After heating, the sample is allowed to cool to room temperature. The fabric "tail" may be blotted to hasten its drying.
5. The sample plate is placed in the testing machine's lower clamp, after first debonding about 0.5" of the bound fabric from the metal plate. The fabric tail is the 3 inches of the fabric strip that are longer than the test panel about 180° from the upper clamp. The fabric "tail" is then placed into the testing machine's upper clamp such that it is bent back upon itself. The initial reading of the force display parameter is zeroed.
6. Tinius Olsen Machine Settings:
   Force = 50% = 1.13 kg (2.50 lbs.)
   Ext. = 100 = 12.7 cm (5.0 in.)
   Speed = 6.35 cm/min. (2.50 in./min.)
7. The Tinius Olsen Testing Machine evenly pulls the fabric strip from the metal plate while simultaneously recording the adhesive force and distance the cross-hair travels.

Data are reported as force per width of adhesive strip (lbs/in). Data collected is between 15-85% of load cell weight.

**Table 1:**

| **Comparison of Adhesive strength for PAE and TGIC resins with and without typical wet end chemicals** | | | |
|---|---|---|---|
| **Creping Aid Chemistry** | **Creping aid** | **W/** **5ppm** **Bleach** | **W/** **100ppm** **Bisulfite** |
| AD-DETA-EPI, Bisulfite | 0.75 | | |
| AD-DETA-TGIC, Bisulfite | 0.68 | | |
| | | | |
| Rezosol 8223® | 0.89 | | |
| CAP-IT-DETA-TGIC-H2SO4, Low MW | 0.81 | | |
| CAP-IT-DETA-TGIC-H2SO4, High MW | 0.92 | | |
| CAP-IT-DETA-TGIC-Bisulfite | 0.71 | | |
| | | | |
| Unisoft 805® | 0.83 | | |
| Unisoft 805A® | 0.62 | | |
| DMG-DETA-TGIC-Bisulfite | 0.59 | | |

### VI. Epichlorohydrin and Byproduct Residuals

The TGIC resins were evaluated for residual epichlorohydrin and epichlorohydrin byproducts. The TGIC resins have substantially lower epichlorohydrin byproducts. The TGIC creping aids and their epichlorohydrin analogs were tested for epichlorohydrin, 1,3-dichloropropanol (1,3-DCP), 2,3-dichloropropanol (2,3-DCP), and 3-chloropropanediol (3-CPD) residual by gas chromatography using a halogen specific detector. As expected, the TGIC resins had a significantly lower level of epichlorohydrin byproducts. The creping aid chemistries included Rezosol® 8223, a polyamidoamine-epichlorohydrin resin made available by Hercules Incorporated as well as Unisoft 805® and Unisoft 805A®, also polyamidoamine-epichlorohydrin resins made available by Hercules Incorporated.

**Table 2:**

| **Comparison of Epichlorohydrin and Epichlorohydrin Byproduct Composition of PAE and TGIC Resins** | | | | |
|---|---|---|---|---|
| **Creping Aid Chemistry** | **Epi (ppm)** | **1,3-DCP (ppm)** | **2,3-DCP (ppm)** | **3-CPD (ppm)** |
| AD-DETA-TGIC, Bisulfite | 75 | 0.05 | 0.07 | 0.11 |
| | | | | |
| Rezosol® 8223 | <5ppm | 5900.00 | <5ppm | 1500 |
| | | | | |
| Unisoft 805® | 75 | 34 | 10 | 132 |
| Unisoft 805A® | 75 | 12 | 61 | 82 |
| DMG-DETA-TGIC-Bisulfite | 75 | <0.05 | 0.08 | 0.06 |

### VII. Pilot Papermachine Trial Using TGIC Resins

In addition, high and low molecular weight (MW) TGIC resins were evaluated on a pilot paper machine. The high molecular weight Caprolactam-Itaconic acid-Diethylenetriamine-TGIC resin trialed on a pilot paper machine produced soft paper as shown in the softness panel test ranking (SPT). Interestingly, the paper produced by the high molecular weight TGIC resin was slightly softer than the PAE standard resin that in tern typically produces softer paper than the Caprolactam-Itaconic acid-DETA-epichlorohydrin resin analog of the TGIC resin.

The TGIC resins were initially developed as creping aids. Two resins made from caprolactam-itaconic acid-diethylenetriamine (CAP-IT-DETA) and TGIC were tested at the pilot paper machine at James River, Neenah Technical Center now part of Georgia Pacific. Also, like the PAE resins, the two TGIC resins were determined not to be primary dermal irritants.

Rezosol® 8223 is a polyamidoamine-epichlorohydrin resin made available by Hercules Incorporated. Hercules 82-176 is also a polyamidoamine-epichlorohydrin resin made available by Hercules Incorporated.

**Table 3:**

| **Pilot Paper Machine Trial of TGIC Resins as Creping Aids** | | | |
|---|---|---|---|
| **Creping Aid** | **Add on (Lbs/Ton)*** | **Release Agent Add On (Lbs/Ton)*** | **SPT** |
| Rezosol 8223® | 0.1 | 1.50 | 17.0 |
| Rezosol 8223® | 0.2 | 1.85 | 17.2 |
| Rezosol 8223® | 0.3 | 2.25 | 17.3 |
| CAP-IT-DETA-TGIC, H2SO4,low MW | 0.1 | 1.50 | 16.8 |
| CAP-IT-DETA-TGIC, H2SO4,low MW | 0.2 | 1.85 | 17.1 |
| CAP-IT-DETA-TGIC, H2SO4,low MW | 0.3 | 2.25 | 17.8 |
| Hercules 82-176 Adhesion Aid | 0.1 | 1.50 | 17.6 |
| Hercules 82-176 Adhesion Aid | 0.2 | 1.85 | 17.8 |
| Hercules 82-176 Adhesion Aid | 0.3 | 2.25 | 17.8 |
| CAP-IT-DETA-TGIC, H2SO4, high MW | 0.1 | 1.50 | 17.7 |
| CAP-IT-DETA-TGIC, H2SO4, high MW | 0.2 | 1.85 | 17.7 |
| CAP-IT-DETA-TGIC, H2SO4, high MW | 0.3 | 2.25 | 17.8 |
| CAP-IT-DETA-TGIC, H2SO4, high MW | 0.1 | 1.50 | 17.6 |
| CAP-IT-DETA-TGIC, H2SO4, high MW | 0.3 | 2.25 | 18.0 |

| | | | |
|---|---|---|---|
| * 1 lbs/ton ≙ 0.5 kg/t | | | |

### VIII. Evaluation of the TGIC as Wet Strength Agents

TGIC crosslinked polyamidoamines have been tested as wet strength resins (WSR) in laboratory handsheets. When sufficient amount of TGIC is used to crosslink polyamidoamines, the resulting TGIC resin is able to impart wet strength into the resulting paper. A series of TGIC crosslinked resin have been prepared and show wet strength agent properties.

### A. Handsheet Preparation and Evaluation

The TGIC resins were evaluated a wet strength agents in the lab by preparing 7.5kg/280m² (16.5 pound/3000 ft²) handsheets. With a furnish of 60% bleached softwood Kraft pulp and 40% chemi-thermo-mechanical-pulp (CTMP), where CMC was used the CMC solution was added after the wet strength resin.

### B. Testing:

The handsheets are placed in a TAPPI conditioning room (standard temperature and humidity) for about 2 weeks. Next the handsheets are cut into 2.54 cm (1") strips in both cross directions and machine direction (CD and MD) and cured in an oven at about 80°C for about 1 hour. Finally, the strips are evaluated for basis weight, dry tensile strength, and wet tensile strength via TAPPI Method T-494. For wet tensile strength the test strips are soaked in water for about one minute prior to testing.

**Table 4:**

| **Wet Strength Properties of TGIC resin, Kymene® 557H, and blank** | | | | | | | |
|---|---|---|---|---|---|---|---|
| **WSR** | **% WSR**^{**1**} | **% CMC**^{**2**} | **CD W/D**^{**3**} | **CD Std Dev**^{**4**} | **MD**^{**5**} **W/D** | **MD**^{**6**} **Std Dev** | **GMT**^{**7**} **@16.5 Lbs/3000 ft**^{**2**}** |
| Blank | 0 | 0 | 0.000 | | 0 | | 0 |
| Kymene® 557H | 1 | 0.2 | 0.275 | 0.042 | 0.251 | 0.040 | 0.263 |
| DMG-DETA-TGIC (1:0.8) | 0.75 | 0 | 0.204 | 0.032 | 0.187 | 0.022 | 0.195 |
| DMG-DETA-TGIC (1:0.8) | 1 | 0 | 0.193 | 0.031 | 0.212 | 0.043 | 0.203 |
| DMG-DETA-TGIC (1:0.8) | 1.25 | 0 | 0.194 | 0.042 | 0.271 | 0.086 | 0.229 |
| DMG-DETA-TGIC (1:0.8) | 0.75 | 0.15 | 0.196 | 0.027 | 0.202 | 0.023 | 0.199 |
| DMG-DETA-TGIC (1:0.8) | 1 | 0.2 | 0.223 | 0.025 | 0.274 | 0.075 | 0.248 |
| DMG-DETA-TGIC (1:0.8) | 1.25 | 0.25 | 0.243 | 0.033 | 0.243 | 0.046 | 0.243 |
| Blank | 0 | 0 | 0.000 | | 0 | | 0 |
| Kymene® 557H | 1 | 0.2 | 0.280 | 0.039 | 0.324 | 0.081 | 0.301 |
| DMG-DETA-TGIC (1:0.8)* | 0.75 | 0.15 | 0.262 | 0.039 | 0.252 | 0.05 | 0.257 |
| DMG-DETA-TGIC (1:0.8)* | 1 | 0.2 | 0.234 | 0.034 | 0.250 | 0.021 | 0.242 |
| DMG-DETA-TGIC (1:0.8)* | 1.25 | 0.25 | 0.243 | 0.038 | 0.206 | 0.031 | 0.223 |
| | | | 0.278 | 0.041 | 0.288 | 0.241 | 0.282 |

| | | | | | | | |
|---|---|---|---|---|---|---|---|
| 1= Wet Strength Resin, | | | | | | | |
| 2= Carboxymethylcellulose, | | | | | | | |
| 3= Cross Direction Wet/Dry Ratio, | | | | | | | |
| 4= Cross Direction Standard Deviation, | | | | | | | |
| 5= Machine Direction Wet/Dry Ratio, | | | | | | | |
| 6= Machine Direction Standard Deviation, | | | | | | | |
| 7= Geometric Mean Tensile | | | | | | | |
| * Activated with NaOH Grand Mean Kymene 557H Pooled Standard Deviation | | | | | | | |
| ** 1 lbs/3000 ft² ≙7.5 kg/280 m² | | | | | | | |

The DMG-DETA-TGIC resin has wet strength performance comparable to Kymene® 557H. Some increase in wet strength performance is seen activating the resin prior to preparing handsheets. The DMG-DETA-TGIC resin was activated by raising the pH of a 3% solids solution to pH 11 with sodium hydroxide. The solution was allowed to activate for about 30 minutes and then was diluted to a solids content of 1%. The activated solution was used to make handsheets within four hours of activation.

## Claims

1. A resin, C, obtainable by reacting a polyamidoamine, A, with a cross-linking agent, K, which is at least one polyepoxide selected from the group consisting of glycerol triglycidyl ether (triglycidyl glycerol), triphenylolmethane triglycidyl ether, trimethylolethane triglycidyl ether, trimethylolpropane triglycidyl ether, 1,2,4-butanetriol triglycidyl ether, 1,2,6-hexanetriol triglycidyl ether, 1,2,3-heptanetriol triglycidyl ether, pentaerythritol triglycidyl ether, 1,1,1-tris(4-hydroxyphenyl)-ethane triglycidyl ether, calix[4]arene triglycidyl ether, calix[6]arene triglycidyl ether, 4-t-butylcalix[4]arene triglycidyl ether, 4-t-butylcalix[6]arene triglycidyl ether, pyrogallol triglycidyl ether, 1,2,4-benzenetriol triglycidyl ether, phloroglucinol triglycidyl ether, and triglycidylisocyanurate.

2. The resin according to claim 1, wherein the mole ratio of reactive functionalities of K:A is at least 1:1.

3. The resin according to claim 1, wherein the polyamidoamine is a member of the group consisting of adipic acid-diethylenetriamine, dimethylglutarate-diethylenetriamine, caprolactam-itaconic acid-diethylenetriamine, caprolactam-itaconic acid-6-aminohexanoic acid-diethylenetriamine, and methylbisaminopropylamine-oxalic acid-urea.

4. The resin according to claim 1, wherein the polyepoxide is triglycidylisocyanurate.

5. The resin according to claim 1, wherein the resin is selected from one of:
A is adipic acid-diethylenetriamine polymer, and K is triglycidylisocyanurate;
A is caprolactam-itaconic acid-diethylenetriamine polymer, and K is triglycidylisocyanurate;
A is caprolactam-itaconic acid-6 aminohexanoic acid-diethylenetriamine polymer, and K is triglycidylisocyanurate;
A is dimethylglutarate-diethylenetriamine polymer, and K is triglycidylisocyanurate;
A is methylbisamino propylamine-oxalic acid-urea polymer, and K is triglycidylisocyanurate.

6. The resin according to claim 1, wherein said resin has a solids content from 10% to 50% by weight based on solids.

7. A process for preparing the resin, C, as defined in any one of claims 1 to 6, comprising reacting the polyamidoamine, A, with the cross-linking agent, K.

8. The process according to claim 7, wherein A is in solution, the solution having a solids content from 30% to 70% by weight based on solids.

9. The process according to claim 7 further comprising quenching the reaction using an acid wherein the resin solution is 15 % to 50% by weight based on solids.

10. The process according to claim 7 further comprising quenching the reaction using a sulfite wherein the resin solution is 10% to 60% by weight based on solids.

11. Use of a resin as claimed in any one of claims 1 to 6 as a wet strength agent and/or creping aid for preparing cellulosic products, as a dye fixative for preparing textiles or cellulosic products, as an additive for preparing cosmetics and hair conditioners, as a crease resistance aid for textiles, as an adhesive for floor tiles or as a backing agent for ceiling tiles.

12. A cellulosic product, textile product, cosmetic product or hair conditioner containing the resin as claimed in any one of claims 1 to 6.

## Patentansprüche

1. Harz (C), erhältlich durch Umsetzen eines Polyamidoamins (A) mit einem Vernetzungsmittel (K), das wenigstens ein Polyepoxid ist, das aus der Gruppe ausgewählt ist, die aus Glycerintriglycidylether (Triglycidylglycerin), Triphenylolmethantriglycidylether, Trimethylolethantriglycidylether, Trimethylolpropantriglycidylether, 1,2,4-Butantrioltriglycidylether, 1,2,6-Hexantrioltriglycidylether, 1,2,3-Heptantrioltriglycidylether, Pentaerythrittriglycidylether, 1,1,1-Tris(4-hydroxyphenyl)-ethantriglycidylether, Calix[4]arentriglycidylether, Calix[6]arentriglycidylether, 4-t-Butylcalix[4]arentriglycidylether, 4-t-Butylcalix[6]arentriglycidylether, Pyrogalloltriglycidylether, 1,2,4-Benzoltrioltriglycidylether, Phloroglucintriglycidylether und Triglycidylisocyanurat besteht.

2. Harz gemäss Anspruch 1, worin das Molverhältnis der reaktiven Funktionalitäten von (K):(A) wenigstens 1:1 ist.

3. Harz gemäss Anspruch 1, worin das Polyamidoamin ein Element aus der Gruppe ist, die aus Adipinsäure-Diethylentriamin, Dimethylglutarat-Diethylentriamin, Caprolactam-Itaconsäure-Diethylentriamin, Caprolactam-Itaconsäure-6-Aminohexansäure-Diethylentriamin und Methylbisaminopropylamin-Oxalsäure-Harnstoff besteht.

4. Harz gemäss Anspruch 1, worin das Polyepoxid Triglycidylisocyanurat ist.

5. Harz gemäss Anspruch 1, worin das Harz aus einem der folgenden ausgewählt ist:
(A) ist Adipinsäure-Diethylentriamin-Polymer und (K) ist Triglycidylisocyanurat;
(A) ist Caprolactam-Itaconsäure-Diethylentriamin-Polymer und (K) ist Triglycidylisocyanurat;
(A) ist Caprolactam-Itaconsäure-6-Aminohexansäure-Diethylentriamin-Polymer und (K) ist Triglycidylisocyanurat;
(A) ist Dimethylglutarat-Diethylentriamin-Polymer und (K) ist Triglycidylisocyanurat;
(A) ist Methylbisaminopropylamin-Oxalsäure-Harnstoff-Polymer und (K) ist Triglycidylisocyanurat.

6. Harz gemäss Anspruch 1, worin das Harz einen Feststoffgehalt von 10 bis 50 Gew.%, bezogen auf Feststoffe, hat.

7. Verfahren zur Herstellung des Harzes (C), wie in einem der Ansprüche 1 bis 6 definiert, umfassend das Umsetzen des Polyamidoamins (A) mit dem Vernetzungsmittel (K).

8. Verfahren gemäss Anspruch 7, worin (A) in Lösung ist, wobei die Lösung einen Feststoffgehalt von 30 bis 70 Gew.%, bezogen auf Feststoffe, hat.

9. Verfahren gemäss Anspruch 7, das ferner das Abschrecken der Reaktion unter Verwendung einer Säure umfasst, worin die Harzlösung 15 bis 50 Gew.%, bezogen auf Feststoffe, ist.

10. Verfahren gemäss Anspruch 7, das ferner das Abschrecken der Reaktion unter Verwendung eines Sulfits umfasst, worin die Harzlösung 10 bis 60 Gew.%, bezogen auf Feststoffe, ist.

11. Verwendung eines Harzes gemäss einem der Ansprüche 1 bis 6 als Nassverfestigungsmittel und/oder Krepphilfsmittel zur Herstellung von Celluloseprodukten, als Farbstoffixiermittel zur Herstellung von Textilien oder Celluloseprodukten, als Additiv zur Herstellung von Kosmetika und Haarspülungsmitteln, als Knitterfestigkeitshilfsmittel für Textilien, als Haftvermittler für Bodenfliesen oder als Rückverstärkungsmittel für Deckenplatten.

12. Celluloseprodukt, Textilprodukt, Kosmetikprodukt oder Haarspülungsmittel, enthaltend das Harz gemäss einem der Ansprüche 1 bis 6.

## Revendications

1. Résine, C, pouvant être obtenue en faisant réagir une polyamidoamine, A, avec un agent de réticulation, K, qui est au moins un polyépoxyde choisi dans le groupe consistant en éther triglycidylique de glycérol (triglycidylglycérol), éther triglycidylique de triphénylolméthane, éther triglycidylique de triméthyloléthane, éther triglycidylique de triméthylolpropane, éther triglycidylique de 1,2,4-butanetriol, éther triglycidylique de 1,2,6-hexanetriol, éther triglycidylique de 1,2,3-heptanetriol, éther triglycidylique de penta-érythritol, éther triglycidylique de 1,1,1-tris(4-hydroxyphényl)-éthane, éther triglycidylique de calix[4]arène, éther triglycidylique de calix[6]arène, éther triglycidylique de 4-tertiobutylcalix[4]arène, éther triglycidylique de 4-tertiobutylcalix[6]arène, éther triglycidylique de pyrogallol, éther triglycidylique de 1,2,4-benzènetriol, éther triglycidylique de phloroglucinol et isocyanurate de triglycidyle.

2. Résine suivant la revendication 1, dans laquelle le rapport molaire des fonctionnalités réactives de K:A est égal à au moins 1:1.

3. Résine suivant la revendication 1, dans laquelle la polyamidoamine est un membre du groupe consistant en acide adipique-diéthylènetriamine, glutarate de diméthyldiéthylènetriamine, caprolactame-acide itaconique-diéthylènetriamine, caprolactame-acide itaconique-acide 6-aminohexanoïque-diéthylènetriamine et méthylbisaminopropylamine-acide oxalique/urée.

4. Résine suivant la revendication 1, dans laquelle le polyépoxyde est l'isocyanurate de triglycidyle.

5. Résine suivant la revendication 1, ladite résine étant choisie entre les résines suivantes :
A représente un polymère acide adipique-diéthylènetriamine et K représente l'isocyanurate de triglycidyle ;
A représente un polymère caprolactame-acide itaconique-diéthylènetriamine et K représente l'isocyanurate de triglycidyle ;
A représente un polymère caprolactame-acide itaconique-acide 6-aminohexanoïque-diéthylènetriamine et K représente l'isocyanurate de triglycidyle ;
A représente un polymère glutarate de diméthyldiéthylènetriamine et K représente l'isocyanurate de triglycidyle ;
A représente un polymère méthylbisaminopropylamineacide oxalique-urée et K représente l'isocyanurate de triglycidyle.

6. Résine suivant la revendication 1, ladite résine ayant une teneur en matières solides de 10 % à 50 % en poids sur la base des matières solides.

7. Procédé pour la préparation de la résine, C, telle que définie dans l'une quelconque des revendications 1 à 6, comprenant la réaction de la polyamidoamine A avec l'agent de réticulation K.

8. Procédé suivant la revendication 7, dans lequel A est en solution, la solution ayant une teneur en matières solides de 30 % à 70 % en poids sur la base des matières solides.

9. Procédé suivant la revendication 7, comprenant en outre la désactivation de la réaction utilisant un acide, la solution de résine étant une solution à une teneur de 15 % à 50 % en poids sur la base des matières solides.

10. Procédé suivant la revendication 7, comprenant en outre la désactivation de la réaction en utilisant un sulfite, la solution de résine étant une solution à une teneur de 10 % à 60 % en poids sur la base des matières solides.

11. Utilisation d'une résine suivant l'une quelconque des revendications 1 à 6 ccmme agent améliorant la résistance à l'état humide et/ou adjuvant de crêpage pour la préparation de produits cellulosiques, comme fixateur de colorant pour la préparation de matières textiles ou de produits cellulosiques, comme additif pour la préparation de produits cosmétiques et d'agents de conditionnement capillaire, comme adjuvant de résistance au froissement pour des matières textiles, comme adhésif pour des dalles de sols ou comme agent de support pour des dalles de plafond.

12. Produit cellulosique, produit textile, produit cosmétique ou agent de conditionnement capillaire contenant la résine suivant l'une quelconque des revendications 1 à 6.
